Europäisches Patentamt

European Patent Office

Office européen des brevets

(19)

(11) Publication number: **0 226 800**
A2

(12)

# EUROPEAN PATENT APPLICATION

(21) Application number: **86115740.2**

(22) Date of filing: **12.11.86**

(51) Int. Cl.⁴: **C 12 N 15/00**
C 12 N 1/20, C 07 K 7/00
C 07 K 7/10, C 12 N 9/52
//(C12N1/20, C12R1:43)

(30) Priority: **15.11.85 JP 257450/85**
**07.02.86 JP 26120/86**
**02.06.86 JP 128921/86**

(43) Date of publication of application:
**01.07.87 Bulletin 87/27**

(84) Designated Contracting States:
**AT BE CH DE ES FR GB GR IT LI LU NL SE**

(71) Applicant: **Takeda Chemical Industries, Ltd.**
**27, Doshomachi 2-chome Higashi-ku**
**Osaka-shi Osaka, 541(JP)**

(72) Inventor: **Kikuchi, Masakazu**
**4-16, Higashitokiwadai 7-chome Toyono-co**
**Toyono-gun Osaka 563-01(JP)**

(72) Inventor: **Nakahama, Kazuo**
**15-59 Nishinokyo Nagaokakyo**
**Kyoto 617(JP)**

(72) Inventor: **Marumoto, Ryuji**
**53-1, Okuikeminami-machi**
**Ashiya Hyogo 659(JP)**

(74) Representative: **von Kreisler, Alek, Dipl.-Chem. et al,**
**Deichmannhaus am Hauptbahnhof**
**D-5000 Köln 1(DE)**

(54) **Novel recombinant gene and use thereof.**

(57) The present invention relates to a DNA encoding ser-rapeptase polypeptide or a polypeptide of similar anti-inflammatory activity. The serrapetase polypeptide or a poly-peptide of similar anti-inflammatory activity can be produced industrially at high efficiency using the DNA.

EP 0 226 800 A2

# Novel Recombinant Gene and Use Thereof

The present invention relates to DNAs encoding serrapeptase (International Nonproprietary Name), which works well as a pharmaceutical etc., or a polypeptide possessing anti-inflammatory activity like that of serrapeptase, and to their uses.

Conventional production methods for serrapeptase, which works well as an anti-inflammatory agent etc., include the method in which a Serratia bacterium is cultured (refer to US Patent No. 3691014). However, as serrapeptase gene has not been cloned, the production of serrapeptase by gene technology have not been available.

It can be expected that in producing serrapeptase by the culturing of a Serratia bacterium, serrapeptase productivity will be improved by gene amplification. In addition, it is thought that the procedure of serrapeptase purification can be simplified by reducing the production of proteins produced simultaneously with serrapeptase by the Serratia bacterium, such as DNase.

The present inventors found that a transformant carrying a plasmid having a cloned DNA containing serrapeptase gene from chromosomal DNA of a serrapeptase-producing Serratia bacterium will produces a notable amount of serrapeptase and a polypeptide possessing anti-inflammatory activity like that of serrapeptase.

The inventors made further studies based on these findings, developing the present invention.

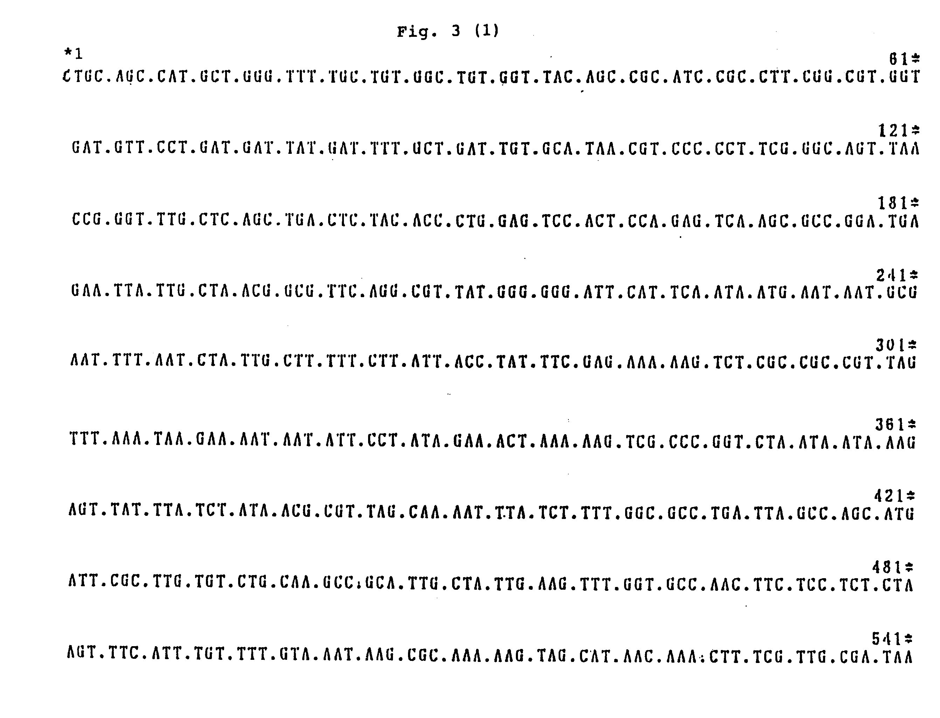

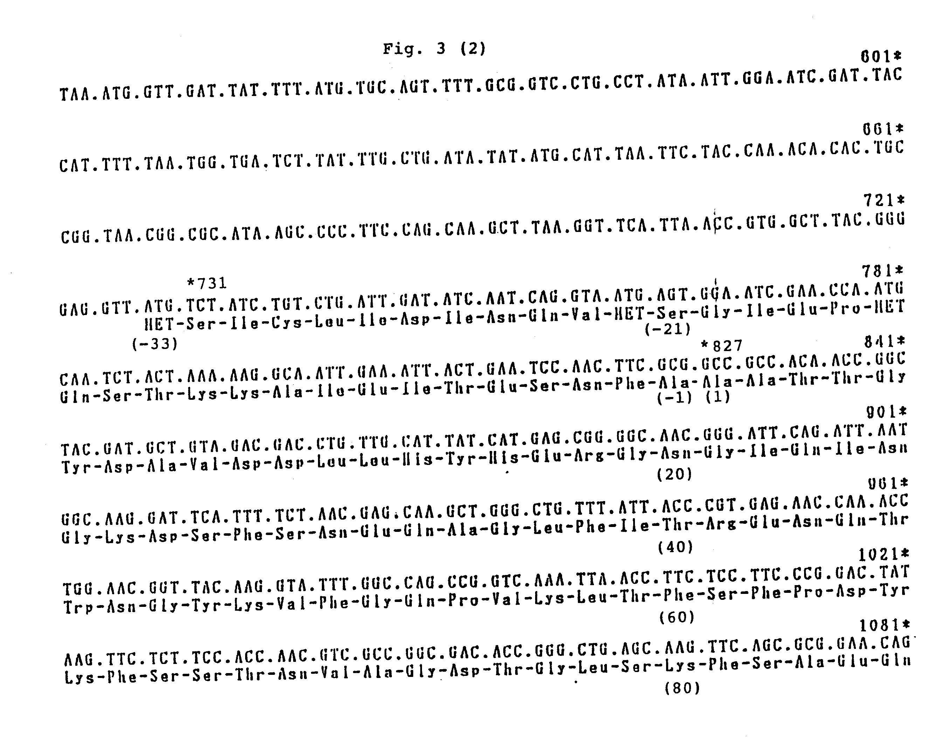

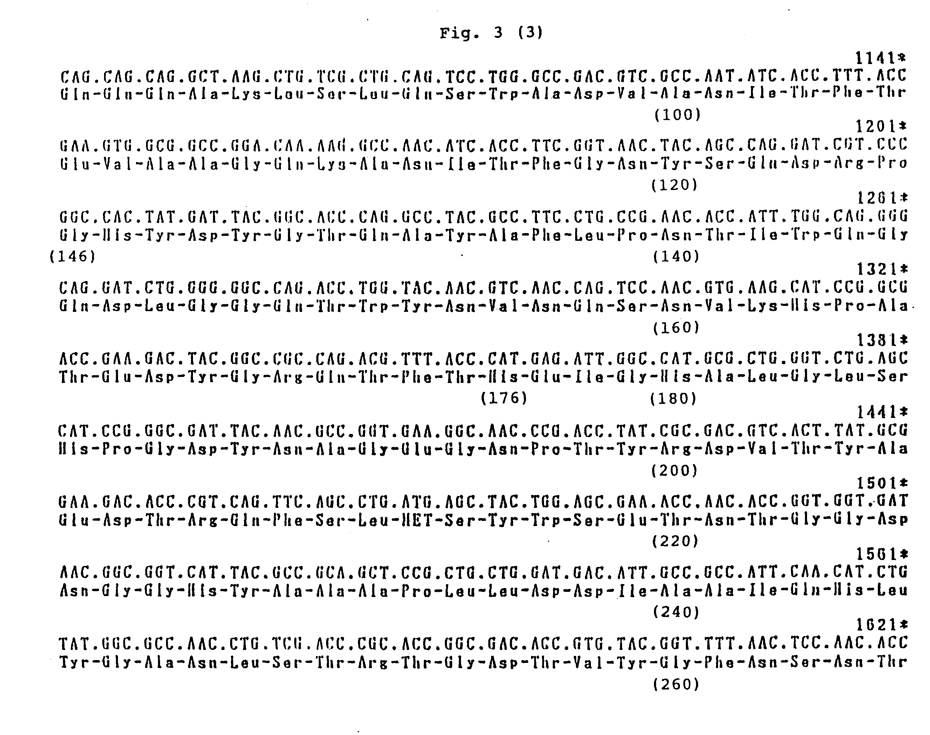

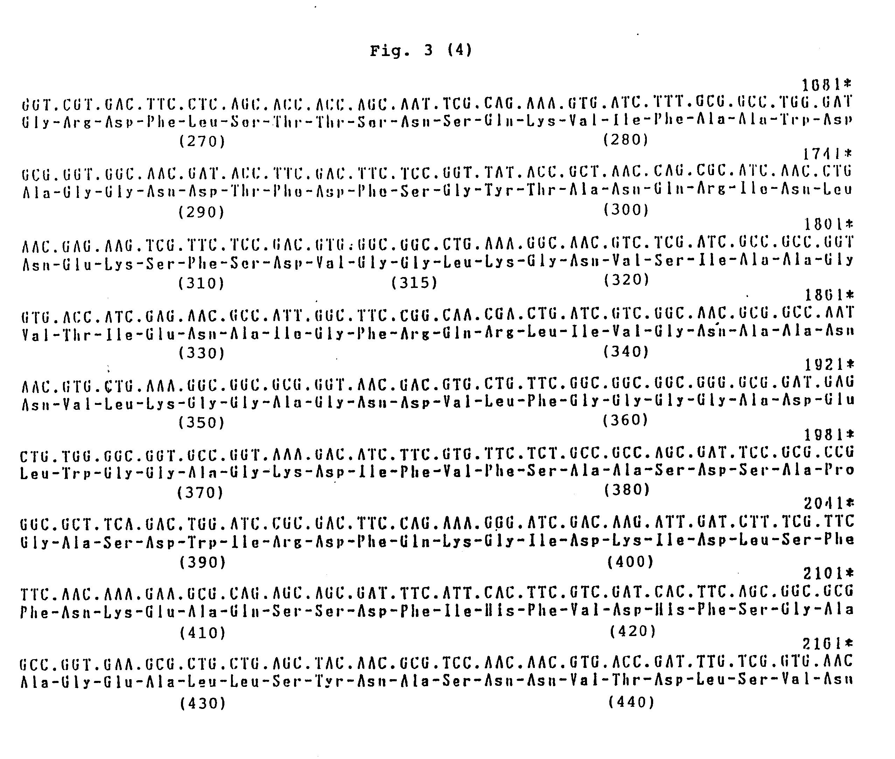

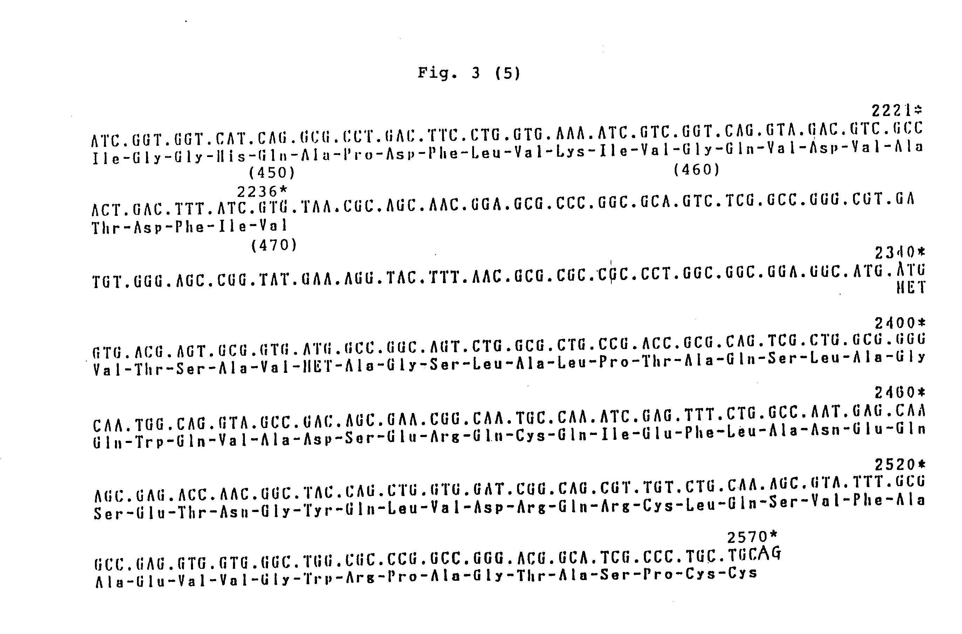

The present invention provides a DNA encoding serrapeptase polypeptide or a polypeptide possessing anti-inflammatory activity like that of serrapeptase, a plasmid containing said DNA, a transformant carrying said plasmid, a polypeptide having the amino acid sequence of No. (1) to No. (470) shown in Figure 3 (VII) which may have Met at N-terminus thereof, and production and use thereof. The present invention further provides a DNA having a base sequence represented by the formula:

```
ATG   TCT   ATC   TGT   CTG   ATT   GAT   ATC   AAT
CAG   GTA   ATG   AGT   GGA   ATC   GAA   CCA   ATG
CAA   TCT   ACT   AAA   AAG   GCA   ATT   GAA   ATT
ACT   GAA   TCC   AAC   TTC   GCG
```

[VIII]

or a part of said base sequence,
a polypeptide represented by the formula:

```
Met-Ser-Ile-Cys-Leu-Ile-Asp-Ile-Asn-Gln-Val-
Met-Ser-Gly-Ile-Glu-Pro-Met-Gln-Ser-Thr-Lys-
Lys-Ala-Ile-Glu-Ile-Thr-Glu-Ser-Asn-Phe-Ala
```

[IX]

a part of said polypeptide, or a polypeptide resulting from either the substitution, insertion, addition or elimination of some amino residues in said polypeptide or said partial polypeptide, and production and use thereof which are advantageously used for the production of recombinant proteins such as protease.

As to the DNA encoding serrapeptase polypeptide or a polypeptide possessing anti-inflammatory activity like that of serrapeptase mentioned above, a DNA, which hybridizes with a DNA whose base sequence is represented by the formula:

$$5'\text{ACTTTGTAX}_1\text{CCGTCCCAX}_2{}^G$$
$$\text{TTTGGTTTTC}^{3'}$$

[I]

wherein $X_1$ and $X_2$ represent either T or G and either T or G, respectively, or a DNA which hybridizes with a DNA whose base sequence is represented by the formula:

$$5' TCY_1 TGY_2 CCY_3 TGCCA 3' \qquad\qquad [II]$$

wherein $Y_1$, $Y_2$, and $Y_3$ represent either T or C, either A, G, T, or C, and either T or C, respectively, may be exemplified.

Also exemplified are a DNA which has a base sequence of No. 731 to No. 2236 shown in Figure 3 having ATG at 5'-terminus and having one or two out of three termination codons TAA, TAG and TGA at 3'-terminus thereof [III], and a DNA which has a base sequence of No. 827 to No. 2236 shown Figure 3 having ATG or hydrogen atom at the 5'-terminus and having one or two out of three termination codons TAA, TAG and TGA at the 3'-terminus thereof (IV). The DNA (IV) may also have base sequences at its 5'- and 3'-terminus as shown in the base sequence of No. 1 to No. 2570 in Figure 3 [V]. These DNA may be a part of said base sequence, or a base sequence resulting from either the substitution, insertion, addition or elimination of some bases in said base sequence or the part thereof.

The above mentioned DNA may encode a polypeptide containing an amino acid sequence of the formula:

His-Glu-Ile-Gly-His-Ala-Leu--35 to 45 amino acid residues--Gly-Asp-Asn-Gly- Gly-His-Tyr--70-80 amino acid residues-- Leu-Asn-Glu-Lys-Ser-Phe-Ser-Asp-Val-Gly-Gly,

or a polypeptide containing an amino acid sequence resulting from either the substitution, insertion, addition or elimination of some amino acid residues in said amino acide sequence.

The substitution, insertion, addition and elimination mentioned above are, for example, the following manner:

Ile being substituted with Leu or Met,
Ile-Gly-His being substituted with Ile-Thr-His,
His-Ala-Leu being substituted with His-Gly-Lue,
His-Ala-Val or His-Gly-Val, Gly-His-Tyr being
substituted with Gly-Val-His-Tyr and Glu-Lys-Ser-Phe
being substituted with Glu-Ser-Phe or Glu-Ser-Ile.

The polypeptide mentioned above may have, for
example an amino acid sequence of No. (176) to No. (315)
shown in Figure 3 or amino acid sequence of No. (146)
to No. (365) shown in Figure 3.

For example, polypeptide having an amino acid
sequence of No. (-33) to No. (470) [VI] or No. (1) to
No. (470) [VII] in shown Figure 3 may be mentioned
as a whole amino acid sequence.

The base sequences expressed by Formula [III] or [V] or their partial sequences can be obtained also by the combination of substitution, insertion, addition, and elimination of bases.

The polypeptides expressed by Formula [VI] or [IX] or their partial polypeptides can be obtained also by the combination of substitution, insertion, addition, and elimination of bases.

It should be noted that in the present specification either serrapeptase or a polypeptide possessing anti-inflammatory activity like that of serrapeptase is designated a "serrapeptase" in some cases.

In addition, the DNA encoding either serrapeptase or a polypeptide possessing anti-inflammatory activity like that of serrapeptase is designated "serrapeptase gene" in some cases.

The DNA encoding either serrapeptase or a polypeptide possessing anti-inflammatory activity like that of serrapeptase can obtained from e.g. the chromosomal DNA of protease-producing bacterium of the genus Serratia.

Serratia strains which can be used include Serratia marcescens. To show concrete examples: Serratia marcescens ATCC21074, S. marcescens IFO3046, S. marcescens IFO3735, etc. can be used.

Said ATCC21074 strain has been registered in the American Type Culture Collection (ATCC) since May 15, 1967, and is listed in the American Type Culture Collection Catalogue of Bacteria, Phages and rDNA Vectors, 16th edition, 1985.

Said IFO3046 and IFO3735 strains are listed in Institute for Fermentation Osaka List of Cultures, 7th edition, 1984, published by the Institute for Fermentation, Osaka (IFO).

The method of preparing chromosomal DNA from Serratia bacterial cells is now in the public domain; it can be carried out by e.g. the method of Lovett et al. [Methods in Enzymology, 68, 342 (1979)].

The chromosomal DNA obtained as described above is then cleaved using a restriction enzyme. Fragments resulting from partial decomposition using a restriction enzyme can also be used as chromosomal DNA.

The resulting chromosomal DNA fragment is then linked to the vector DNA using DNA ligase.

Vectors which are used most commonly include ColE1-derived plasmids pBR322 [refer to Gene, 2, 95 (1977)], pBR325 [refer to Gene, 4, 121 (1978)], pUC12 [Vieira, J. and Messing, J., refer to Gene, 19, 259 (1982)], and pUC13 [refer to Gene, 19, 259 (1982)]; however, any other plasmids can also be used, as long as their replication is maintained within the host.

Said linking is carried out as follows: The vector DNA is first cleaved with a restriction enzyme. In general, the use of a restriction enzyme which cleaves the vector DNA at one point is recommended. Said cleavage can be carried out by a conventional method.

Linking said chromosomal DNA fragment to the vector DNA using DNA ligase can be carried out by a conventional method.

The desired recombinant DNA is then selected out of the recombinant DNAs obtained as described above. Any host-vector systems can be used, as long as the presence of the serrapeptase gene is detected on the chromosomal DNA fragment by transformation following the linking of the chromosomal DNA fragment to the vector. For example, the Escherichia coli host-vector system can be used. To describe more concretely: Escherichia coli is transformed in accordance with a conventional method, e.g., the method described in the Proceedings of the National Academy of Sciences of the United States of America (hereinafter abbreviated Proc. Nat. Acad. Sci. U.S.A. in some cases), 69, 2110 (1972), using a recombinant DNA obtained by linking the chromosomal DNA fragment to the vector obtained as described above. Transformant selection can be carried out on the basis of the drug resistances of the plasmid used. For example, either ampicillin resistance or tetracycline resistance can be used as the selection criterion when pBR322 is used as the vector.

In addition, the desired clone can be selected out of the previously obtained drug-resistant transformants via secondary screening by a conventional method, e.g. the colony hybridization method [Grunstein-Hogness's method: Proc. Nat. Acad. Sci. U.S.A. 72, 3961 (1975)] using a $^{32}$P-labeled, chemically synthesized oligonucleotide having a base sequence regarded as corresponding to the amino acid sequence of a serrapeptase whose amino acid sequence is already known as a probe.

DNAs which can be used as probes include a DNA whose base sequence is the invert of that deduced from the amino acid sequence of Serratia protease between the 42nd and 51st positions (Glu-Asn-Gln-Thr-Trp-Asp-Gly-Tyr-Lys-Val) reported by Lee, I. S. et al. [Federation Proceedings, 44, March p. 1057 (1985)], represented by the general formula:

$$5'A\ C\ T\ T\ T\ G\ T\ A\ X_1\ C\ C\ G\ T\ C\ C\ C\ A\ X_2\ G$$

$$T\ T\ T\ G\ G\ T\ T\ T\ T\ C\ 3' \qquad [I]$$

[In this formula, $X_1$ and $X_2$ represent either T or G and either T or G, respectively.] and a DNA whose base sequence is the invert of that deduced from said amino acid sequence of Serratia protease between the 143rd and 147th positions (Trp-Gln-Gly-Gln-Asp), represented by the general formula:

$$5'T\ C\ Y_1\ T\ G\ Y_2\ C\ C\ Y_3\ T\ G\ C\ C\ A\ 3' \qquad [II]$$

[In this formula, $Y_1$, $Y_2$, and $Y_3$ represent either T or C, either A, G, T, or C, and either T or C, respectively.].

The DNA represented by Formula [I] and that represented by Formula [II] can be used in combination.

The base sequence of the DNA cloned by colony hybridization using these probes is determined either by the Maxam-Gilbert method [Proc. Nat. Acad. Sci. U.S.A., 74, 560 (1977)]or the dideoxy method [Messing, J. et al.; Nucleic Acids Research, 9, 309 (1981)] and compared with known amino acid sequences to detect the serrapeptase gene. When the region encoding the serrapeptase gene is not covered entirely, re-cloning should be carried out using the cloned fragment obtained from the positive clone as a new prove to prepare a DNA fragment containing the entire serrapeptase gene encoding region.

Plasmid extraction from the transformant obtained as described above is carried out by e.g. the method described in Nucleic Acids Research, 7, 1513 (1979) to obtain the desired plasmid in which a DNA encoding either serrapeptase or a polypeptide possessing anti-inflammatory activity like that of serrapeptase is integrated.

Specifically, the DNA having the base sequence represented by Formula [V] can be produced by partially decomposing a plasmid, e.g. pTSP25, with the restriction enzyme PstI, as shown in Examples of prefered embodiment.

The DNAs having the base sequences represented respectively by Formulas [III], [IV], and [VIII] can be produced by the conventional method of fragment cleavage with a restriction enzyme and fragment ligation with ligase and if necessary, in combination with the use of a chemically synthesized DNA fragment, using said plasmid as the material. It is also possible to synthesize the whole sequence chemically.

The site-directed mutagenesis method [Methods in Enzymology, Vol. 100, p. 468 (1983), Academic Press N.Y.] is used commonly to either substitute, insert, and, or eliminate at least one base in a base sequence.

The DNA encoding either the polypeptide represented by Formula [VI], a part of said polypeptide, or a polypeptide resulting from either the substitution, insertion, addition, or elimination of at least one amino residue in said polypeptide or said partial polypeptide can also be produced by the method mentioned above.

The resulting DNA encoding either serrapeptase or a polypeptide possessing anti-inflammatory activity like that of serrapeptase can be used as the structural gene for the production of either serrapeptase or a polypeptide possessing anti-inflammatory activity like that of serrapeptase.

In addition, the DNA encoding either the polypeptide represented by Formula [VI], a part of said polypeptide, or a polypeptide resulting from either the substitution, insertion, addition, or elimination of at least one amino residue in said polypeptide or said partial polypeptide, obtained as described above can be used as the structural gene for the production of said polypeptide.

A transformant is then produced using the plasmid in which the DNA encoding either serrapeptase or a polypeptide possessing anti-inflammatory activity like that of serrapeptase, obtained as described above.

The following can be used as host for such transformant production: microbes such as Escherichia bacteria, Serratia bacteria, Bacillus bacteria and yeasts; and eucaryotic cells such as animal cells.

Escherichia bacteria which can be used as hosts include Escherichia coli, to describe concretely, E. coli DH1 strain [refer to Nature, 217, 1110 (1968)], E. coli JM103 strain [refer to Nucleic Acids Research, 9, 309 (1981)], E. coli JA221 strain [refer to the Journal of Molecular Biology, 120, 517 (1978)], and E. coli RRI strain [refer to Methods in Enzymology, 68, 262 (1979)].

Serratia bacteria which can be used as host include Serratia marcescens, to describe concretely, S. marcescens IFO3759, S. marcescens ATCC21074, S. marcescens IFO3046, S. marcescens IFO3735, S. marcescens No. 87, and S. marcescens Nc-4.

Said Serratia marcescens IFO3759 strain has been deposited at the Institute for Fermentation, Osaka (IFO) since September 23, 1958, and is listed in the Institute for Fermentation List of Cultures, 7th edition, 1984, published by the institute. Its bacteriological characteristics are the same as those described in the Journal of Bacteriology, 11, 76-77 (1926).

The bacteriological characteristics of said Serratia marcescens ATCC21074 strain is described in US Pat. No. 3691014.

Said Serratia marcescens No. 87 strain and S. marcescens NC-4 strain have been deposited at the Institute for Fermentation Osaka (IFO) under the deposit numbers IFO14454 and IFO14504 since July 5, 1985 and April 17, 1986, respectivley; these microbes have been deposited at the Fermentation Research Institute (FRI), Agency of Industrial Science and Technology, MITI, Japan under the deposit numbers FERM BP-1181 and FERM BP-1182 since November 7, 1985 and May 29, 1986 under Budapest Treaty, respectively.

The bacteriological characteristics of said Serratia marcescens No. 87 and S. marcescens NC-4 are the same as those of S. marcescens described in Bergey's Manual of Determinative Bacteriology, 8th edition.

Bacillus bacteria which can be used as hosts include Bacillus subtilis, to describe concretely, Bacillus subtilis MI 114 [Gene, 24, 255 (1983)].

Yeasts which can be used as hosts include Saccharomyces cerevisiae AH22R⁻ [Proc. Nat. Acad. Sci. USA, 80, 1 (1983)].

Animal cells which can be used as hosts include the mouse L-cell, the Chinese hamster ovary (CHO) cell, and the COS cell.

Methods in the public domain can be used to transform said Escherichia or Serratia bacteria, e.g. the methods described in the Proc. Nat. Acad. Sci. USA, 69, 21110 (1972), Gene, 17, 107 (1982), etc.

Said <u>Bacillus</u> bacteria can also be transformed in accordance with methods in the public domain, such as the methods described in Molecular and General Genetics <u>168</u>, 111 (1979) etc.

The transformation methods for eucaryotic cells such as yeasts and animal cells are also in the public domain, e.g., the method described in US Pat. No. 4399216 can be used.

Moreover, serrapeptase expressivity can be improved by introducing to the host the entire serrapeptase gene or its part cleaved off from the obtained clone after being linked downstream in an appropriate promoter sequence. Where necessary, a Shine-Dalgarno sequence (SD sequence) can be inserted downstream of the promoter.

When either <u>Escherichia</u> <u>coli</u> or a <u>Serratia</u> bacterium is used as the host, it is recommended that the tryptophane (trp) promoter, bacteriophage-derived PL promoter, lactose (lac) promoter, protein chain elongation factor Tu (tufB) promoter, recA promoter, etc. be used as promoters.

When a <u>Bacillus</u> bacterium is used as the host, the use of SPO1 promoter, veg promoter, $P_1$ promoter [refer to EP Publication (laid open) No. 0146901], etc. is recommended.

When a yeast is used as the host, PhO5 promoter, GLD promotor, PGK promoter, ADH promoter, PHO81 promoter, etc. can be used.

Also when a eucaryotic cell such as an animal cell is used as the host, any promoter can be used, as long as it is suitable to the host species.

As described above, transformants were obtained by tansforming a host with a plasmid having the DAN encoding a serrapeptase.

When the resulting transformant is either a bacterium (<u>Escherichia</u>, <u>Serratia</u>, <u>Bacillus</u>) or a yeast, it is possible to produce a serrapeptase by culturing the transformant in a medium so that the serrapeptase is accumulated in the medium.

When a transformant is cultured whose host is a bacterium, the use of a liquid medium is recommended, which should contain a carbon source, a nitrogen source, minerals and other substances essential to the growth of the transformant. Substances which can be used as carbon sources include glucose, dextrin, soluble starch and sucrose; substances which can be used as nitrogen sources include ammonium salts, nitrates, corn steep liquor, peptone, casein, meat extract, soybean cake and other organic or inorganic substances; minerals which can be used include calcium chloride, monobasic sodium phosphate and magnesium chloride. Yeast extract, vitamins, growth promoting factors etc. may also be added. The use of a culture medium containing approx. 0.1% dihydrated calcium chloride and approx. 2% dihydrated monobasic sodium phosphate is particularly favorable for protease production. Culture conditions such as temperature, pH and duration should be chosen so that the production and activity of the desired serrapeptase are at their highest. In general, it is preferable that culture temperature be about 25 ∿ 35°C for Serratia bacteria or about 20 ∿ 40°C for Escherichia or Bacillus bacteria, that culture pH be very slightly acidic to very slightly alkaline (near neutral is preferable) and that culture duration be about 2 days.

When a transformant whose host is a yeast is cultured, the Burkholder minimum medium [Bostian, K. L. et al., Proc. Nat. Adad. Sci. USA, 77, 4505 (1980)] can be used. Culture temperature should be about 15 ∿ 45°C, preferably 24 ∿ 37°C; culture time should be about 10 ∿ 96 hours, preferably 24 ∿ 72 hours, with aeration or stirring if necessary.

Transformant obtained using as host a eucaryotic cell, such as an animal cell, should be made to produce a serrapeptase by culturing in accordance with a conventional method.

0226800

Culture media which can be used include Eagle's medium [H. Eagle; Science, 130, 432 (1959)], Dulbecco's modified Eagle's medium [Orgad Laub and William J. Rutter; Journal of Biological Chemistry, 258, 6043 (1983)] and HAT medium [Littlefield, J. W.; Science, 145, 709 (1964)].

Culturing should be at 30 ∿ 42°C, preferably 35 ∿ 37°C, for about 1 ∿ 10 days.

The serrapeptase accumulated in the cells is extracted by an ordinary method, e.g. the ultrasonic disintegration method, the disintegration method using a French press, mechanical disintegration such as grinding, enzymatic disintegration using lysozyme etc., or the chemical extraction method using guanidine hydrochloride etc. The resulting extract or supernatant is then separated and purified by a purification method in the public domain, e.g. precipitation using a precipitator, isoelectric precipitation, salting-out, dialysis, adsorption using an adsorbent such as ion exchange resin, gel filtration or high performance liquid chromatography.

That is, the liquid containing a serrapeptase obtained via the present invention can be precipitated either by salting-out with sodium sulfate, ammonium sulfate, common salt etc. or by adding an appropriate hydrophilic organic solvent such as an alcohol or acetone. Since the serrapeptases obtained by the present invention are stable, it is possible to concentrate, under reduced pressure, liquid containing the serrapeptase in low concentration, such as culture liquid. Other purification methods which can be employed include adsorption/desorption using calcium phosphate, alumina, bentonite, ion exchange resin etc.; chromatography; precipitation using a protein precipitator such as nucleic acid, tannic acid or phosphorus wolframate; impurity separation/removal using a heavy metal salt; isoelectric precipitation and electrodialysis.

It is preferable that the vector obtained by the present invention have an inserted DNA region encoding the signal peptide which will cause extracellular secretion of the produced serrapeptase, so that the desired serrapeptase is accumulated outside the cultured transformant cells for easy purification. Any DNA regions enclosing such a signal sequence can be used for the secretion, as long as they are suitable to the host.

In addition, the use of a transformant other than a Serratia strain is recommended, because neither DNase nor other undesired protein is produced in the culture medium during culturing, thus the desired serrapeptase can be obtained easily.

When Serratia strain, known to produce serrapeptase, are used as the hosts for the transformants described above, their serrapeptase productivity can be improved by using the present invention.

As described above, serrapeptases, which work well as an anti-inflammatory agent etc., can be produced at high industrial efficiency using the transformants involved in the present invention.

Serrapeptase and polypeptides possessing anti-inflammatory activity similar to of serrapeptase, produced by the method involved in the present invention, can be used for example as anti-inflammatory agents in the same manner as described in US Pat. No. 3691014.

Either the polypeptides represented by Formula [IV], their parts, or polypeptides resulting from either the substitution, insertion, addition, or elimination of at least one amino residue in said polypeptides can be produced by a method like that for serrapeptases. This production method has some advantages, as has the production method for serrapeptase.

Since either the polypeptides represented by Formula [VI], their parts, or the polypeptides resulting from either the substitution, insertion, addition, or elimination of at least one amino residue possess anti-inflammatory activity like that of serrapeptase, they can be used as anti-inflammatory agents in the same manner as serrapeptase.

It is expected that DNAs whose base sequence is either that represented by Formula [VIII] or a part thereof will be used as genes to encode signal peptides in the field of gene engineering. It is therefore possible to make a foreign gene secrete its products outside the cell by linking the gene downstream of said DNA.

It is also expected that the polypeptides represented by Formula [IX], their parts, or the polypeptides resulting from either the substitution, insertion, addition, or elimination of at least one amino residue in said polypeptides will be used as signal peptides in gene engineering.

The abbreviations for bases, amino acids etc., used in the present specification are based either on the abbreviations specified by the IUPAC-IUB Commission on Biochemical Nomenclature or those used commonly in related fields, as shown below. It should be noted that when there is a possibility of the presence of an optical isomer in amino acids, the isomer is an L-body unless otherwise stated.

| DNA: | Deoxyribonucleic acid | Gln: | Glutamine |
| A : | Adenine | Glu: | Glutamic acid |
| C : | Cytosine | Gly: | Glycine |
| G : | Guanine | His: | Histidine |
| T : | Thymine | Ile: | Isoleucine |
| Ala: | Alanine | Leu: | Leucine |
| Arg: | Arginine | Lys: | Lysine |
| Asn: | Asparagine | Met: | Methionine |
| Asp: | Aspartic acid | Phe: | Phenylalanine |
| Cys: | Cystein | Pro: | Proline |

Ser:  Serine            Tyr:  Tyrosine
Thr:  Threonine         Val:  Valine
Trp:  Tryptophane

Brief Explanation of the Drawings

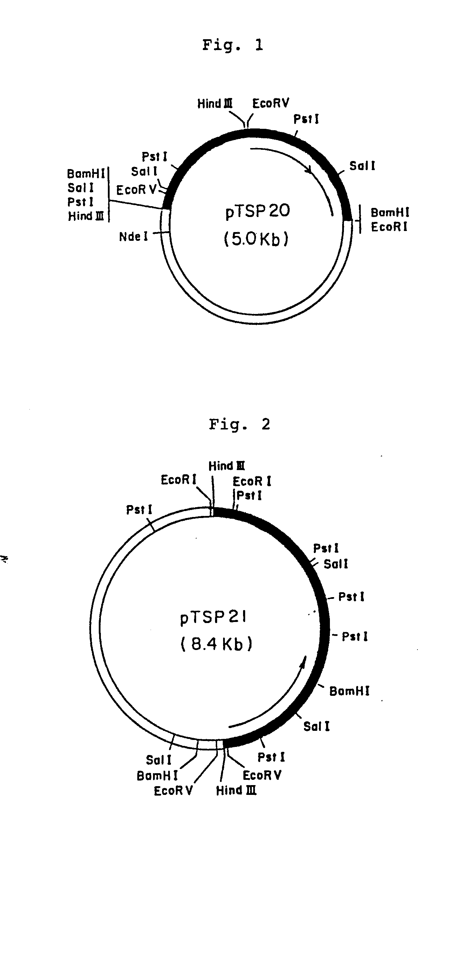

Figures 1 to 6 respectively show the restriction map for the plasmid pTSP20 obtained in Example 2, the restriction map for the plasmid pTSP21 obtained in Example 4, the base sequence of the serrapeptase-encoding gene obtained in Example 5 and the amino acid sequence deduced from the base sequence, the restriction map for the plasmid pTSP25 obtained in Example 6, the restriction map for pTSP26 obtained in Example 6, and the restriction map for pTSP31 obtained in Example 8.

Examples

The present invention is hereinafter described more concretely with some examples of its preferred embodiments.

Example 1

Isolation of Chromosomal DNA of <u>Serratia</u> <u>marcescens</u>:

<u>Serratia</u> <u>marcescens</u> ATCC 21074 was grown at 28°C for 16 hours in a 200-mℓ conical flask containing 40mℓ of an L-medium (pH 7.3) composed of 1% Tripton (Difco Laboratories, USA), 0.5% yeast extract (Difco Laboratories), 0.5% NaCℓ and 0/1% glucose. 1mℓ of the resulting culture liquid was inoculated to 40mℓ of an L-medium in a 200-mℓ conical flask and cultured at 37°C for 2 hours with shaking.

Bacterial cells were collected from the culture (10mℓ) by centrifugation and washed twice with 40mM tris HCℓ (pH 8.5) - 25mM EDTA. The washed cells were then suspended in 1mℓ 40mM tris·HCℓ (pH 8.5) - 25mM EDTA to which 0.1mℓ of a 10mg/mℓ lysozyme solution was added, after which the suspension was incubated at 37°C for 20 minutes. The suspension was then added with 1mℓ of a mixture of 0.75% Salcosyl-40mM tris·HCℓ (pH 8.5) - 25mM EDTA and incubated at 65°C for 8 minutes, after which 0.1mℓ of a 5mg/mℓ ribonuclease solution was added and the suspension was incubated at 37°C for 30 minutes. 0.1mℓ of a 10mg/mℓ pronase solution was further added before incubation at 37°C for 1 hour. After extraction with phenol, DNA was precipitated with ethanol. The resulting precipitate was then dissolved in 0.5mℓ of a mixture of 10mM tris·HCℓ (pH 8.0) - 1mM EDTA (TE buffer solution) and dialyzed against the same buffer solution at 4°C for 2 days to yield a DNA solution.

Example 2

Cloning of Serrapeptase-encoding Gene:

(A) Preparation of gene library

1.0µg of a plasmid (pUC12) was digested posed with 10 units of the restriction enyzme BamHI at 37°C for 2 hours and then treated with 0.1 unit of alkali phosphatase (Washington Inc., USA) at 65°C for 30 minutes. After phenol and ether extraction, the DNA was precipitated with ethanol.

Separately, 3µg of the chromosomal DNA of Serratia marcescens ATCC 21074, obtained in Example 1, was digested with 10 units of the restriction enzyme BamHI at 37°C for 1 hour, after which it was treated at 65°C for 15 minutes and precipitated by ethanol.

The resulting BamHI-decomposed pUC12 (10ng) and BamHI-decomposed chromosomal DNA (40ng) were each dissolved in water and combined together, after which they were reacted with each other at 15°C for 16 hours in a liquid (10µℓ) containing 10nM ATP, 20 units of T4DNA ligase (New England Biolabs, USA) and a ligase buffer solution. Using the resulting reaction liquid, either Escherichia coli DH1 or E. coli JM103 was transformed in accordance with the method described in the Proc. Nat. Adad. Sci. USA, 69, 2110 (1972), yielding a large number of ampicillin-resistant colonies.

(B) Preparation of probes

On the basis of the amino acid sequences of Serratia protease reported by Lee, I. S. et al. [Federation Proceedings, 44, March p. 1057 (1985)], DNA with a base sequence the invert of that deduced from amino acids at positions 42 ∿ 51 (Glu-Asn-Gln-Thr-Trp-Asp-Cly-Tyr-Lys-Val), expressed by General Formula [I], (a mixture of 4 compounds) and DNA with a base sequence the invert of that deduced from amino acids at positions 143 ∿ 147 (Trp-Gln-Gly-Gln-Asp), expressed by General Formula [II], (a mixture of 16 compounds), respectively, were chemically synthesized in accordance with the report by Crea, R et al. [Proc. Nat. Acad. Sci. USA, 75, 5765 (1978)] for use as probes.

2μg of each chemically synthesized oligonucleotide was incubated at 37°C for 10 minutes in 50μℓ of a liquid containing 50mM tris HCℓ (pH 7.6), 10mM MgCℓ$_2$, 10mM mercaptoethanol, 40μCiγ-$^{32}$PATP and 12 units of T4 polynucleotide kinase, after which it was reacted with 2μℓ of 0.1mM ATP at 37°C for 20 minutes to label its 5' terminal with $^{32}$P.

(C) Screening by colony hybridization

The DNAs of the about 700 ampicillin-resistant, tetracycline-sensitive colonies obtained in Example 2 (A) were each fixed on a nitrocellulose filter using Grunstein-Hogness's method [Proc. Nat. Acad. Sci. USA, 72, 3961 (1975)].

The fixed DNAs were then hybridized with the probes prepared in Example 2 (B) in accordance with the method of Lawn et al. [Nucleic Acids Research, 9, 6103 (1981)], after which one clone positive to both probes was selected by autoradiography. A plasmid DNA was isolated from this clone using Brinboim-Doly's method [Nucleic Acids Research, 7, 1513 (1979)], and designated pTSP20. Figure 1 shows the restriction map of this plasmid.

In Figure 1, ▭ and ■■ indicate the DNA fragment derived from the plasmid pUC12 and that derived from Serratia marcescens ATCC 21074 chromosome, respectively.

Example 3

Determination of base sequence:

The 0.42kb HindIII-PstI fragment isolated from pTSP20 was inserted into the phages M13mp8 and M13mp9 in accordance with the method of Messing et al. [Nucleic Acids Research, 9, 309 (1981)] and its base sequence was determined by the dideoxy method [Sanger et al.; Proc. Nat. Acad. Sci. USA, 74, 5463 (1977)].

A part of nucleotide sequence determined as described above is as follows:

Table 1

```
G C C G C C A C A A C C G G C T A C G A T
Ala  Ala  Thr  Thr  Gly  Tyr  Asp
(1)                 (5)

G C T G T A G A C G A C C T G T T G C A T
Ala  Val  Asp  Asp  Leu  Leu  His
          (10)

T A T C A T G A G C G G G G C A A C
Tyr  His  Glu  Arg  Gly  Asn
(15)                      (20)
```

The amino acid sequence deduced from this base sequence completely coincides with that of Serratia protease between the 1st and 20th positions, as reported by Lee et al. [Federation Proceedings, 44, March p. 1057 (1985)]. It is therefore clear that pTSP20 has a gene encoding serrapeptase.

Example 4

Re-cloning of serrapeptase-encoding gene:

The serrapeptase-encoding gene cloned in Example 2 lacked the region encoding the C-terminal. The serrapeptase gene was re-cloned to obtain the region.

The plasmid pBR322 (1µg) was digested with 5 units of the restriction enzyme HindIII at 37°C for 1 hour and treated with 0.1 unit of alkaline phosphatase (Washington Inc., USA) at 65°C for 30 minutes; after phenol extraction and ether extraction, it was precipitated by ehtanol.

Chromosomal DNA of Serratia marcescens ATCC 21074 (3µg) obtained in Example 1 was digested with 10 units of the restriction enzyme Hind at 37°C for 1 hour, treated at 65°C for 15 minutes and precipitated with ethanol.

The HindIII-digested pBR 322 (10ng) and the HindIII-digested chromosomal DNA (50ng) were dissolved in water and combined, after which reaction was carried out in a liquid (10µℓ) containing 10nM ATP, 20 units of T4 DNA ligase (New England Biolabs, USA) and a ligase buffer solution at 15°C for 16 hours. Using the resulting reaction liquid, Escherichia coli DH1 was transformed according to the method described in the Proc. Nat. Acad. Sci. USA, 69, 2110 (1972), yielding a total of 6000 transformant colonies on agar plates containing ampicillin (100µg/mℓ).

The colonies were then transferred onto nitrocellulose filters (Schreicher & Schul, USA) by placing the filters onto the agar plates; filters and plates were then incubated at 37°C for 4 hours with the colony-side uppermost. The filters onto which the colonies grew were detached from the plates, left in air on filter papers saturated with 0.5N NaOH for 10 minutes, placed on filter papers saturated with 1M tris HCℓ (pH 7.5) - 1.5M NaCℓ, left in air for 5 minutes, dried in an air blow for 30 ∿ 60 minutes, and heated at 78°C for 3 hours to fix DNA on the filters.

The region encoding the amino acids of serrapeptase at positions 93 ∿ 391 is present on the 0.9kb PstI-BamHI fragment in the pTSP20.

This fragment was labeled by nick translation in accordance with the method of Maniatis et al. [Molecular Cloning, A Laboratory Manual (Cold Spring Harbor Laboratory, 1982)] and used as a probe to clone the serrapeptase-encoding gene by colony hybridization. As a result, 4 candidate colonies were obtained; the plasmid isolated from one of them was designated pTSP21. Figure 2 shows its restriction map. In Figure 2, ⬜ and ⬛ indicate the DNA fragment derived from pBR322 and that derived from Serratia marcescens ATCC 21074 chromosome, respectively.

Example 5

Determination of base sequence of serrapeptase gene:

The 2kb PstI-BamHI fragment, one of the DNA fragments of pTSP20 derived from Serratia marcescens ATCC 21074 chromosome, obtained in Example 2, and the 1.85kb HindIII-PstI fragment, one of the DNA fragments of pTSP21 derived from Serratia marcescens ATCC 21074 chromosome, obtained in Example 4, were cleaved using various restriction enzymes. The resulting small fragments were inserted into the phage M13 in accordance with the methods of Messing et al. [Nucleic Acids Research, 9, 309 (1981)]; their base sequences were determined by the dideoxy method [Sanger et al.; Proc. Nat. Acad. USA, 74, 5463 (1977)]. Figure 3 shows the base sequence of a DNA containing the serrapeptase gene and the amino acid sequence deduced from the base sequence. As shown in Figure 3, the amino acid sequence of the mature protein of the serrapeptase completely coincides with the amino acid sequence of Serratia protease determined using the Edman method by Lee et al. [Federation Proceedings, 44, March p. 1057 (1985)].

Example 6

Construction of plasmid containing entire serrapeptase gene:

The plasmid pTSP20 lacks the region encoding the C-terminal portion of serrapeptase; pTSP 21 lacks part of the promoter region of the serrapeptase gene. Using these two plasmids, the serrapeptase expression plasmid was constructed as follows:

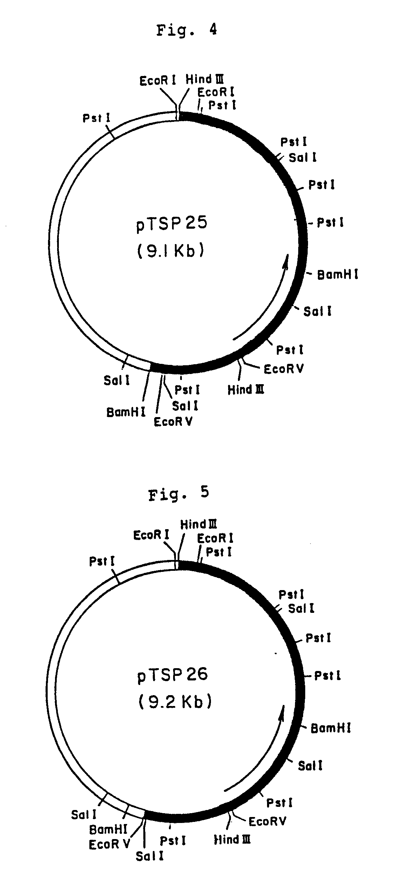

(1) The 6.8kb BamHI fragment isolated from pTSP21 was treated with alkaline phosphatase and ligated to the 2.3kb BamHI fragment isolated from pTSP 20, after which Escherichia coli DH1 and E. coli JM 103 were transformed using the ligated fragments to yield ampicillin-resistant transformants. A plasmid isolated from one of them was designated pTSP25. Figure 4 shows the restriction map for this plasmid. In Figure 4, ▭ and ■ respectively indicate the DNA fragment derived from the plasmid pBR322 and that derived from Serratia marcescens ATCC 21074 chromosome.

(2) After pTSP21 was digested with the restriction enzyme EcoRV, it was treated with alkaline phosphatase and ligated, to the 1.0kb EcoRV fragment isolated from pTSP20; after which Escherichia coli DH1 and E. coli JM103 were transformed using the ligated fragments. A plasmid isolated from one of the ampicillin-resistant transformants obtained as above was designated pTSP26. Figure 5 shows the restriction map for this plasmid. In Figure 5, ▭ and ■ respectively indicate the DNA fragment derived from the plasmid pBR322 and that from Serratia marcescens ATCC 21074 chromosome.

Example 7

Production method for transformants of Serratia bacteria:

Using either the plasmid pTSP21 obtained in Example 4 or the plasmid pTSP25 or pTSP26 obtained in Example 6, Serratia marcescens IFO 3759, S. marcescens No. 87 (IFO 14454, FERM P-8510), S. marcescens ATCC 21074, and S. marcescens NC-4 were transformed in accordance with the method described in Gene, 17, 107 (1982) to yield the following transformants: Serratia marcescens IFO 3759/pTSP21, S. marcescens IFO 3759/pTSP25, S. marcescens IFO 3759/pTSP26, S. marcescens No. 87/pTSP21, S. marcescens No. 87/pTSP25, S. marcescens No. 87/pTSP26, S. marcescens ATCC 21074/pTSP21, S. marcescens ATCC 21074/pTSP25, S. marcescens ATCC 21074/ pTSPS26, S. marcescens NC-4/PTSP21, S. marcescens NC-4/pTSP25, and S. marcescens NC-4/pTSP26.

Example 8

Construction of serrapeptase expression plasmid:

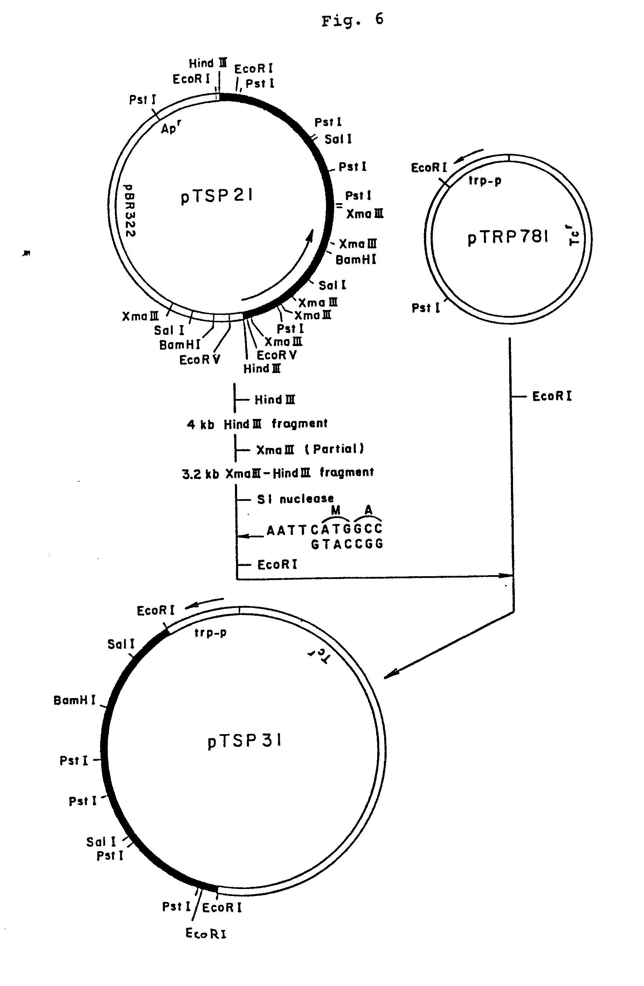

There is a cleavage site for the restriction enzyme XmaIII near the sequence encoding the 1st amino acid (alanine) of the mature protein of serrapeptase. Based on this fact, the 4.0kb HindIII fragment isolated from pTSP21 was partially cleaved with the restriction enzyme XmaIII to yield the 3.2kb XamIII-HindIII fragment, eliminating the 5'-teminal region upstream from the mature protein coding region. The resulting fragment was then treated with S1 nuclease to make both ends flush. The synthetic nucleotides AATTCATGGCC and GGCCATG, with a phosphorylated 5'-terminal, were ligated to the modified fragment, after which the fragment was treated with EcoRI to modify both ends to EcoRI sites. Separately, the expression plasmid pTRP781, having a trp promoter (refer to the Gazette of Jap. Pat. Publication No. 5096/1986), was cleaved with EcoRI; the EcoRI fragments obtained above were ligated to yield the plasmid pTSP31, the direction of whose serrapeptase gene is toward the trp promoter. Figure 6 shows the restriction map for this plasmid. In Figure 6, ⊂⊐ and ▬ respectively indicate the DNA fragment derived from the plasmid pTSP 781 and that derived from Serratia marcescens ATCC 21074 chromosome.

Example 9

Production method for <u>Escherichia coli</u> transformants:

Using the plasmid pTSP21 obtained in Example 4, the plasmids pTSP25 and pTSP26 obtained in Example 6 and the plasmid pTSP31 obtained in Example 7, <u>Escherichia coli</u> DH1 and <u>E. coli</u> JM103 were transformed in accordance with the method described in the Proc. Nat. Acad. Sci. USA, <u>69</u>, 2110 (1972) and Molecular Cloning, A Laboratory Manual (Maniatis et al.; Cold Spring Harbor Laboratory, 1982) to yield the following transformants; <u>Escherichia coli</u> DH1/ pTSP21, <u>E. coli</u> DH1/pTSP25, <u>E. coli</u> DH1/pTSP26, <u>E. coli</u> DH1/pTSP31, <u>E. coli</u> JM103/pTSP21, <u>E. coli</u> JM103/pTSP25, <u>E. coli</u> JM103/pTSP26, and <u>E. coli</u> JM103/pTSP31.

Example 10

Expression of the serrapeptase gene:

The transformant <u>Escherichia coli</u> JM103/pTSP31 obtained in Example 9 was inoculated to a test tube containing 5mℓ brain-heart infusion medium (BHI medium, Difco Laboratories) supplemented with ampicillin (100µg/mℓ) and grown at 37°C for 6 hours with shaking; 0.5mℓ of the culture was transferred to a 200-mℓ conical flask containing 20mℓ BHI medium and grown at 28°C for one day with shaking.    The culture was centrifuged to collect bacterial cells; the cells were washed twice with 50mM tris·HCℓ (pH 8.0) - 50mM NaCℓ and frozen with dry ice-ethanol  The frozen cells were suspended in 2mℓ of 50mM tris·HCℓ (pH 8.0) - 50mM NaCℓ and disrupted by ultrasonication at 19.5kHz for 2 minutes, after which they were centrifuged to prepare their extract.

To 0.2mℓ of the resulting extract 0.3mℓ cold acetone was added, and the mixture centrifuged.  The precipitate, after the addition of 120µℓ of 125mM tris·HCℓ (pH 6.8)-2% SDS-20% glycerol-0.002% bromophenol blue-10% 2-mercaptoethanol, was heated at 100°C for 10 minutes and

centrifuged. The supernatant (30μℓ) was subjected to 10% acrylamide gel electrophoresis (SDS-PAGE). Using an electrophoretic transblotter (Bio-Rad Laboratories), protein was transferred from the acrylamide gel to a nitrocellulose filter, after which serrapeptase content was determined via an Immune Blot Assay System (Bio-Rad Laboratories) using rabbit anti-serrapeptase anti-serum and peroxidase conjugate goat anti-rabbit IgG (Bio-Rad Laboratories): it was found that 250 μg serrapeptase was produced per 1ℓ culture. The product obtained as described above reacts with anti-serrapeptase antibody and exhibits the same behavior in SDS-PAGE as that of standard serrapeptase; based on thses findings, the obtained product is regarded as serrapeptase as concerns its immunological properties and molecular weight.

Example 11

Expression of the Serratia protease gene in Serratia marcescens:

Serratia marcescens NC-4 was transformed via the calcium chloride method [Proc. Nat. Acad. Sci. USA, 69, 2110 (1972)] using the plasmid pTSP26 obtained in Example 6, yielding an ampicillin-resistant transformant.

Serratia marcescens NC-4 and the transformant Serratia marcescens NC-4/pTSP26 were grown at 30°C for 48 hours in a 200-mℓ conical flask containing 20mℓ brain-heart infusion medium with shaking. The cultures were centrifuged; the protease activity of the supernatants was determined [Agric. Biol. Chem., 28, (1964)]. Table 1 shows the results of the determination. The produced protease, after SDS electrophoresis, exhibited a positive response to anti-serrapeptase antibody.

Table 1

| Bacterial Strain | Klett | Protease Production* (mg/ℓ) |
|---|---|---|
| Serratia marcescens NC-4 | 700 | 3.0 |
| Serratia marcescens NC-4/pTSP26 | 700 | 7.5 |

\* Mean value for two experiments.

Based on these results, it was proven that this transformant produced 4.5mg/ℓ Serratia protease in the meidum.

What is claimed is:

1. A DNA encoding serrapeptase polypeptide or a polypeptide possessing anti-inflammatory activity like that of serrapeptase.

2. A DNA according to claim 1, which hybridizes with a DNA whose base sequence is represented by the formula:

$$5'\text{ACTTTGTAX}_1\text{CCGTCCCAX}_2\text{G}$$
$$\text{TTTGGTTTTC}^{3'} \qquad [I]$$

wherein $X_1$ and $X_2$ represent either T or G and either T or G, respectively.

3. A DNA according to claim 1 or 2, which hybridizes with a DNA whose base sequence is represented by the formula:

$$5'\text{TCY}_1\text{TGY}_2\text{CCY}_3\text{TGCCA}^{3'} \qquad [II]$$

wherein $Y_1$, $Y_2$, and $Y_3$ represent either T or C, either A, G, T, or C, and either T or C, respectively.

4. A DNA according to claim 1, which has a base sequence of No. 731 to No. 2236 shown in Figure 3 having ATG at the 5'-terminus and having one or two out of three termination codons TAA, TAG and TGA at the 3'-terminus thereof (III), a part thereof, or a base sequence resulting from either the substitution, insertion, addition or elimination of some bases in said base sequence or the part thereof.

5. A DNA according to claim 1, which has a sequence of No. 827 to No. 2236 shown Figure 3 having ATG or hydrogen atom at the 5'-terminus and having one or two out of three termination codons TAA, TAG and TGA at the 3'-terminus thereof (IV).

6. A DNA according to claim 1, which has a base sequence of No. 1 to No. 2570 shown in Figure 3 [V], a part thereof, or a base sequence resulting from either the substitution, insertion, addition or elimination of some bases in said base sequence or the part thereof.

7. A DNA according to claim 1, which encodes a polypeptide containing an amino acid sequence of the formula:

His-Glu-Ile-Gly-His-Ala-Leu--35 to 45 amino acid residues--Gly-Asp-Asn-Gly-Gly-His-Tyr--70-80 amino acid residues--Leu-Asn-Glu-Lys-Ser-Phe-Ser-Asp-Val-Gly-Gly.

8. A DNA according to claim 7, wherein the polypeptide contains an amino acid sequence resulting from either the substitution, insertion, addition or elimination of some amino acid residues in said amino acide sequence.

9. A DNA according to claim 7, wherein Ile is substituted with Leu or Met.

10. A DNA according to claim 7, wherein Ile-Gly-His is substituted with Ile-Thr-His.

11. A DNA according to claim 7, wherein His-Ale-Leu is substituted with His-Gly-Leu, His-Ala-Val or His-Gly-Val.

12. A DNA according to claim 7, wherein Gly-His-Tyr is substituted with Gly-Val-His-Tyr.

13. A DNA according to claim 7, wherein Glu-Lys-Ser-Phe is substituted with Glu-Ser-Phe or Glu-Ser-Ile.

14. A DNA according to claim 7, wherein the polypeptide contains an amino acid sequence of No. (176) to No. (315) shown in Figure 3.

15. A DNA according to claim 7, wherein the polypeptide contains an amino acid sequence of No. (146) to No. (365) shown in Figure 3.

16. A DNA according to claim 1, wherein the polypeptide has an amino acid sequence of No. (-33) to No. (470) [VI] shown in Figure 3, a part of said polypeptide, or a polypeptide resulting from either the substitution, insertion, addition or elimination of some amino residues in said polypeptide or said partial polypeptide.

17. A DNA according to claim 1, wherein the polypeptide has an amino acid sequence of No. (1) to No. (470) shown in Figure 3 [VII] which may have Met at N-terminus thereof.

18. A DNA having a base sequence represented by the formula:

```
ATG   TCT   ATC   TGT   CTG   ATT   GAT   ATC   AAT
CAG   GTA   ATG   AGT   GGA   ATC   GAA   CCA   ATG
CAA   TCT   ACT   AAA   AAG   GCA   ATT   GAA   ATT
```

```
ACT   GAA   TCC   AAC   TTC   GCG
                                        [VIII]
```

or a part of said base sequence.

19.   A plasmid having a DNA encoding serrapeptase polypeptide or a polypeptide possessing anti-
      inflammatory activity like that of serrapaptase.

20.   A transformant carrying a plasmid having a DNA
      encoding serrapeptase polypeptide or a peptide
      possessing anti-inflammatory activity like that of
      serrapeptase.

21.   A polypeptide which has the amino acid sequence of
      No. (1) to No. (470) shown in Figure 3 (VII) which
      may have Met at N-terminus thereof, a part of said
      polypeptide, or a polypeptide possessing anti-
      inflammatory activity, which results from either
      the substitution, insertion, addition or elimination of some amino residues in said polypeptide or
      said partial polypeptide.

22.   A polypeptide represented by the formula

```
Met-Ser-Ile-Cys-Leu-Ile-Asp-Ile-Asn-Gln-
Val-Met-Ser-Gly-Ile-Glu-Pro-Met-Gln-Ser-
Thr-Lys-Lys-Ala-Ile-Glu-Ile-Thr-Glu-Ser-
Asn-Phe-Ala                         [IX]
```

      a part of said polypeptide, or a polypeptide
      resulting from either the substitution, insertion,
      addition or elimination of some amino residues in
      said polypeptide or said partial polypeptide.

23.   A method for producing serrapeptase polypeptide,
      or a polypeptide possessing anti-inflammatory activity like that of serrapeptase, which comprises growing a transformant carrying a plasmid having a DNA encoding serrapeptase polypeptide possessing anti-inflammatory activity like that of serrapeptase in a medium to produce and accumulate in the culture serrapeptase polypeptide or a polypeptide possessing anti-inflammatory activity like that of serrapeptase and then recovering the same.

1/8

Fig. 1

Fig. 2

Fig. 3 (1)

*1
CTGC.AGC.CAT.GCT.GGG.TTT.TGC.TGT.GGC.TGT.GGT.TAC.AGC.CGC.ATC.CGC.CTT.CGG.CGT.GGT

GAT.GTT.CCT.GAT.GAT.TAT.GAT.TTT.GCT.GAT.TGT.GCA.TAA.CGT.CCC.CCT.TCG.GGC.AGT.TAA

CCG.GGT.TTG.CTC.AGC.TGA.CTC.TAC.ACC.CTG.GAG.TCC.ACT.CCA.GAG.TCA.AGC.GCC.GGA.TGA

GAA.TTA.TTG.CTA.ACG.GCG.TTC.AGG.CGT.TAT.GGG.GGG.ATT.CAT.TCA.ATA.ATG.AAT.AAT.GCG

AAT.TTT.AAT.CTA.TTG.CTT.TTT.CTT.ATT.ACC.TAT.TTC.GAG.AAA.AAG.TCT.CGC.CGC.CGT.TAG

TTT.AAA.TAA.GAA.AAT.AAT.ATT.CCT.ATA.GAA.ACT.AAA.AAG.TCG.CCC.GGT.CTA.ATA.ATA.AAG

AGT.TAT.TTA.TCT.ATA.ACG.CGT.TAG.CAA.AAT.TTA.TCT.TTT.GGC.GCC.TGA.TTA.GCC.AGC.ATG

ATT.CGC.TTG.TGT.CTG.CAA.GCC.GCA.TTG.CTA.TTG.AAG.TTT.GGT.GCC.AAC.TTC.TCC.TCT.CTA

AGT.TTC.ATT.TGT.TTT.GTA.AAT.AAG.CGC.AAA.AAG.TAG.CAT.AAC.AAA.CTT.TCG.TTG.CGA.TAA

Fig. 3 (2)

601*
TAA.ATG.GTT.GAT.TAT.TTT.ATG.TGC.AGT.TTT.GCG.GTC.CTG.CCT.ATA.ATT.GGA.ATC.GAT.TAC

661*
CAT.TTT.TAA.TGG.TGA.TCT.TAT.TTG.CTG.ATA.TAT.ATG.CAT.TAA.TTC.TAC.CAA.ACA.CAC.TGC

721*
CGG.TAA.CGG.CGC.ATA.AGC.CCC.TTC.CAG.CAA.GCT.TAA.GGT.TCA.TTA.AGC.GTG.GCT.TAC.GGG

*731                                                                      781*
GAG.GTT.ATG.TCT.ATC.TGT.CTG.ATT.GAT.ATC.AAT.CAG.GTA.ATG.AGT.GGA.ATC.GAA.CCA.ATG
        MET-Ser-Ile-Cys-Leu-Ile-Asp-Ile-Asn-Gln-Val-MET-Ser-Gly-Ile-Glu-Pro-MET
(-33)                                                           (-21)
                                                    *827              841*
CAA.TCT.ACT.AAA.AAG.GCA.ATT.GAA.ATT.ACT.GAA.TCC.AAC.TTC.GCG.GCC.GCC.ACA.ACC.GGC
Gln-Ser-Thr-Lys-Lys-Ala-Ile-Glu-Ile-Thr-Glu-Ser-Asn-Phe-Ala-Ala-Ala-Thr-Thr-Gly
                                                           (-1) (1)
                                                                     901*
TAC.GAT.GCT.GTA.GAC.GAC.CTG.TTC.CAT.TAT.CAT.GAG.CGG.GGC.AAC.GGG.ATT.CAG.ATT.AAT
Tyr-Asp-Ala-Val-Asp-Asp-Leu-Leu-His-Tyr-His-Glu-Arg-Gly-Asn-Gly-Ile-Gln-Ile-Asn
                                                  (20)
                                                                     961*
GGC.AAG.GAT.TCA.TTT.TCT.AAC.GAG.CAA.GCT.GGG.CTG.TTT.ATT.ACC.CGT.GAG.AAC.CAA.ACC
Gly-Lys-Asp-Ser-Phe-Ser-Asn-Glu-Gln-Ala-Gly-Leu-Phe-Ile-Thr-Arg-Glu-Asn-Gln-Thr
                                                  (40)
                                                                     1021*
TGG.AAC.GGT.TAC.AAG.GTA.TTT.GGC.CAG.CCG.GTC.AAA.TTA.ACC.TTC.TCC.TTC.CCG.GAC.TAT
Trp-Asn-Gly-Tyr-Lys-Val-Phe-Gly-Gln-Pro-Val-Lys-Leu-Thr-Phe-Ser-Phe-Pro-Asp-Tyr
                                                  (60)
                                                                     1081*
AAG.TTC.TCT.TCC.ACC.AAC.GTC.GCC.GGC.GAC.ACC.GGG.CTG.AGC.AAG.TTC.AGC.GCG.GAA.CAG
Lys-Phe-Ser-Ser-Thr-Asn-Val-Ala-Gly-Asp-Thr-Gly-Leu-Ser-Lys-Phe-Ser-Ala-Glu-Gln
                                                  (80)

Fig. 3 (3)

1141*

CAG.CAG.CAG.GCT.AAG.CTG.TCG.CTG.CAG.TCC.TGG.GCC.GAC.GTC.GCC.AAT.ATC.ACC.TTT.ACC
Gln-Gln-Gln-Ala-Lys-Leu-Ser-Leu-Gln-Ser-Trp-Ala-Asp-Val-Ala-Asn-Ile-Thr-Phe-Thr
(100)

1201*

GAA.GTG.GCG.GCC.GGA.CAA.AAG.GCC.AAC.ATC.ACC.TTC.GGT.AAC.TAC.AGC.CAG.GAT.CGT.CCC
Glu-Val-Ala-Ala-Gly-Gln-Lys-Ala-Asn-Ile-Thr-Phe-Gly-Asn-Tyr-Ser-Gln-Asp-Arg-Pro
(120)

1261*

GGC.CAC.TAT.GAT.TAC.GGC.ACC.CAG.GCC.TAC.GCC.TTC.CTG.CCG.AAC.ACC.ATT.TGG.CAG.GGG
Gly-His-Tyr-Asp-Tyr-Gly-Thr-Gln-Ala-Tyr-Ala-Phe-Leu-Pro-Asn-Thr-Ile-Trp-Gln-Gly
(146)                                                        (140)

1321*

CAG.GAT.CTG.GGG.GGC.CAG.ACC.TGG.TAC.AAC.GTC.AAC.CAG.TCC.AAC.GTG.AAG.CAT.CCG.GCG
Gln-Asp-Leu-Gly-Gly-Gln-Thr-Trp-Tyr-Asn-Val-Asn-Gln-Ser-Asn-Val-Lys-His-Pro-Ala
(160)

1381*

ACC.GAA.GAC.TAC.GGC.CGC.CAG.ACG.TTT.ACC.CAT.GAG.ATT.GGC.CAT.GCG.CTG.GGT.CTG.AGC
Thr-Glu-Asp-Tyr-Gly-Arg-Gln-Thr-Phe-Thr-His-Glu-Ile-Gly-His-Ala-Leu-Gly-Leu-Ser
(176)              (180)

1441*

CAT.CCG.GGC.GAT.TAC.AAC.GCC.GGT.GAA.GGC.AAC.CCG.ACC.TAT.CGC.GAC.GTC.ACT.TAT.GCG
His-Pro-Gly-Asp-Tyr-Asn-Ala-Gly-Glu-Gly-Asn-Pro-Thr-Tyr-Arg-Asp-Val-Thr-Tyr-Ala
(200)

1501*

GAA.GAC.ACC.CGT.CAG.TTC.AGC.CTG.ATG.AGC.TAC.TGG.AGC.GAA.ACC.AAC.ACC.GGT.GGT.GAT
Glu-Asp-Thr-Arg-Gln-Phe-Ser-Leu-MET-Ser-Tyr-Trp-Ser-Glu-Thr-Asn-Thr-Gly-Gly-Asp
(220)

1561*

AAC.GGC.GGT.CAT.TAC.GCC.GCA.GCT.CCG.CTG.CTG.GAT.GAC.ATT.GCC.GCC.ATT.CAA.CAT.CTG
Asn-Gly-Gly-His-Tyr-Ala-Ala-Ala-Pro-Leu-Leu-Asp-Asp-Ile-Ala-Ala-Ile-Gln-His-Leu
(240)

1621*

TAT.GGC.GCC.AAC.CTG.TCG.ACC.CGC.ACC.GGC.GAC.AGC.GTG.TAC.GGT.TTT.AAC.TCC.AAC.ACC
Tyr-Gly-Ala-Asn-Leu-Ser-Thr-Arg-Thr-Gly-Asp-Thr-Val-Tyr-Gly-Phe-Asn-Ser-Asn-Thr
(260)

Fig. 3 (4)

1681*

GGT.CGT.GAC.TTC.CTC.AGC.ACC.ACC.AGC.AAT.TCG.CAG.AAA.GTG.ATC.TTT.GCG.GCC.TGG.GAT
Gly-Arg-Asp-Phe-Leu-Ser-Thr-Thr-Ser-Asn-Ser-Gln-Lys-Val-Ile-Phe-Ala-Ala-Trp-Asp
  (270)                                                      (280)

1741*

GCG.GGT.GGC.AAC.GAT.ACC.TTC.GAC.TTC.TCC.GGT.TAT.ACC.GCT.AAC.CAG.CGC.ATC.AAC.CTG
Ala-Gly-Gly-Asn-Asp-Thr-Phe-Asp-Phe-Ser-Gly-Tyr-Thr-Ala-Asn-Gln-Arg-Ile-Asn-Leu
  (290)                                                      (300)

1801*

AAC.GAG.AAG.TCG.TTC.TCC.GAC.GTG.GGC.GGC.CTG.AAA.GGC.AAC.GTC.TCG.ATC.GCC.GCC.GGT
Asn-Glu-Lys-Ser-Phe-Ser-Asp-Val-Gly-Gly-Leu-Lys-Gly-Asn-Val-Ser-Ile-Ala-Ala-Gly
  (310)                      (315)              (320)

1861*

GTG.ACC.ATC.GAG.AAC.GCC.ATT.GGC.TTC.CGG.CAA.CGA.CTG.ATC.GTC.GGC.AAC.GCG.GCC.AAT
Val-Thr-Ile-Glu-Asn-Ala-Ile-Gly-Phe-Arg-Gln-Arg-Leu-Ile-Val-Gly-Asn-Ala-Ala-Asn
  (330)                                                      (340)

1921*

AAC.GTG.CTG.AAA.GGC.GGC.GCG.GGT.AAC.GAC.GTG.CTG.TTC.GGC.GGC.GGC.GGG.GCG.GAT.GAG
Asn-Val-Leu-Lys-Gly-Gly-Ala-Gly-Asn-Asp-Val-Leu-Phe-Gly-Gly-Gly-Gly-Ala-Asp-Glu
  (350)                                                      (360)

1981*

CTG.TGG.GGC.GGT.GCC.GGT.AAA.GAC.ATC.TTC.GTG.TTC.TCT.GCC.GCC.AGC.GAT.TCC.GCG.CCG
Leu-Trp-Gly-Gly-Ala-Gly-Lys-Asp-Ile-Phe-Val-Phe-Ser-Ala-Ala-Ser-Asp-Ser-Ala-Pro
  (370)                                                      (380)

2041*

GGC.GCT.TCA.GAC.TGG.ATC.CGC.GAC.TTC.CAG.AAA.GGG.ATC.GAC.AAG.ATT.GAT.CTT.TCG.TTC
Gly-Ala-Ser-Asp-Trp-Ile-Arg-Asp-Phe-Gln-Lys-Gly-Ile-Asp-Lys-Ile-Asp-Leu-Ser-Phe
  (390)                                                      (400)

2101*

TTC.AAC.AAA.GAA.GCG.CAG.AGC.AGC.GAT.TTC.ATT.CAC.TTC.GTC.GAT.CAC.TTC.AGC.GGC.GCG
Phe-Asn-Lys-Glu-Ala-Gln-Ser-Ser-Asp-Phe-Ile-His-Phe-Val-Asp-His-Phe-Ser-Gly-Ala
  (410)                                                      (420)

2161*

GCC.GGT.GAA.GCG.CTG.CTG.AGC.TAC.AAC.GCG.TCC.AAC.AAC.GTG.ACC.GAT.TTG.TCG.GTG.AAC
Ala-Gly-Glu-Ala-Leu-Leu-Ser-Tyr-Asn-Ala-Ser-Asn-Asn-Val-Thr-Asp-Leu-Ser-Val-Asn
  (430)                                                      (440)

Fig. 3 (5)

2221÷
ATC.GGT.GGT.CAT.CAG.GCG.CCT.GAC.TTC.CTG.GTG.AAA.ATC.GTC.GGT.CAG.GTA.GAC.GTC.GCC
Ile-Gly-Gly-His-Gln-Ala-Pro-Asp-Phe-Leu-Val-Lys-Ile-Val-Gly-Gln-Val-Asp-Val-Ala
(450)                                                    (460)

2236*
ACT.GAC.TTT.ATC.GTG.TAA.CGC.AGC.AAC.GGA.GCG.CCC.GGC.GCA.GTC.TCG.GCC.GGG.CGT.GA
Thr-Asp-Phe-Ile-Val
(470)

2340*
TGT.GGG.AGC.CGG.TAT.GAA.AGG.TAC.TTT.AAC.GCG.CGC.CGC.CCT.GGC.GGC.GGA.GGC.ATG.ATG
                                                                            MET

2400*
GTG.ACG.AGT.GCG.GTG.ATG.GCC.GGC.AGT.CTG.GCG.CTG.CCG.ACC.GCG.CAG.TCG.CTG.GCG.GGG
Val-Thr-Ser-Ala-Val-MET-Ala-Gly-Ser-Leu-Ala-Leu-Pro-Thr-Ala-Gln-Ser-Leu-Ala-Gly

2460*
CAA.TGG.CAG.GTA.GCC.GAC.AGC.GAA.CGG.CAA.TGC.CAA.ATC.GAG.TTT.CTG.GCC.AAT.GAG.CAA
Gln-Trp-Gln-Val-Ala-Asp-Ser-Glu-Arg-Gln-Cys-Gln-Ile-Glu-Phe-Leu-Ala-Asn-Glu-Gln

2520*
AGC.GAG.ACC.AAC.GGC.TAC.CAG.CTG.GTG.GAT.CGG.CAG.CGT.TGT.CTG.CAA.AGC.GTA.TTT.GCG
Ser-Glu-Thr-Asn-Gly-Tyr-Gln-Leu-Val-Asp-Arg-Gln-Arg-Cys-Leu-Gln-Ser-Val-Phe-Ala

2570*
GCC.GAG.GTG.GTG.GGC.TGG.CGC.CCG.GCC.GGG.ACG.GCA.TCG.CCC.TGC.TGCAG
Ala-Glu-Val-Val-Gly-Trp-Arg-Pro-Ala-Gly-Thr-Ala-Ser-Pro-Cys-Cys

022680

Fig. 4

EcoR I  Hind III
         EcoR I
Pst I     Pst I
              Pst I
              Sal I
              Pst I
              Pst I
pTSP25
(9.1 Kb)
              BamHI
              Sal I
              Pst I
              EcoRV
Sal I         Hind III
BamHI  Pst I
       Sal I
EcoRV

Fig. 5

EcoR I  Hind III
         EcoR I
Pst I     Pst I
              Pst I
              Sal I
              Pst I
              Pst I
pTSP26
(9.2 Kb)
              BamHI
              Sal I
              Pst I
              EcoRV
Sal I         Hind III
BamHI
EcoR V  Pst I
        Sal I

Fig. 6

# DECLARATION PURSUANT TO RULE 28, PARAGRAPH 4, OF THE EUROPEAN PATENT CONVENTION

The applicant has informed the European Patent Office that, until the publication of the mention of the grant of the European patent or until the date on which the application has been refused or withdrawn or is deemed to be withdrawn, the availability of the micro-organism(s) identified below, referred to in paragraph 3 of Rule 28 of the European Patent Convention, shall be effected only by the issue of a sample to an expert.

## IDENTIFICATION OF THE MICRO-ORGANISMS

Accession numbers of the deposits:

ATCC 21074
IFO 14454
IFO 14504
FERM BP-1181
FERM BP-1182

EPO Form 1165 07.81 e